# FASCICULE DE BREVET EUROPEEN

(11) **EP 2 031 362 B1**
(45) Date de publication et mention de la délivrance du brevet: **16.11.2011**
(21) Numéro de dépôt: 08163149.1
(22) Date de dépôt: 28.08.2008
(51) Int. Cl.: G01L 1/20

(54) **Capteur de détection et de mesure de pressions incorporant au moins une cellule résistive de détection de forces**
Sensor zur Druckerfassung und messung, der mindestens eine Widerstandszelle zur Kräfteerfassung einschließt
Sensor for detecting and measuring pressure including at least one force-detecting resistive cell

(30) Priorité: 30.08.2007 FR 0757268
(43) Date de publication de la demande: 04.03.2009
(73) Titulaire: Hill-Rom Industries SA, 34100 Montpellier (FR)
(72) Inventeur: Caminade, Jean-Luc M., 34430, SAINT JEAN DE VEDAS (FR); Basilio, Jean-Marie, 34140, MEZE (FR); Coupard, Olivier M., 34090, MONTPELLIER (FR)
(74) Mandataire: Domange, Maxime

(56) Documents cités:
- EP-A- 1 821 088
- WO-A-98/25112
- DE-A1- 3 011 266
- US-A- 6 009 580
- US-A- 6 109 117
- US-A1- 2003 200 611
- US-A1- 2006 070 456
- US-B1- 6 386 051

## Description

La présente invention concerne le domaine de la mesure de pressions et se rapporte plus particulièrement à un capteur de détection et de mesure de pressions comportant au moins une cellule résistive de détection de forces.

Une application privilégiée du capteur de la présente invention concerne en particulier la détection et la mesure de pressions appliquées par le corps de patients alités ou assis sur des dispositifs de support médicalisés comme des matelas thérapeutiques, et notamment les dispositifs de support de type matelas ou coussins à cellules gonflables, afin de réguler la pression de gonflage des cellules du dispositif de support pour lutter contre les pathologies cutanées liées à une immobilisation prolongée sur un lit ou un fauteuil.

On sait en effet dans la pratique médicale que les pressions d'interface entre le corps des patients et leur dispositif de support constituent le facteur principal de développement de complications cutanées, notamment d'escarres de décubitus, dus à l'immobilité prolongée des patients sur leur lit ou leur fauteuil.

Une des techniques éprouvées pour lutter contre la formation et le développement d'escarres chez les patients consiste à supporter les patients sur des lits comportant des matelas à cellules gonflables dont la pression de gonflage est régulée en fonction de la morphologie et de la masse du patient dans le but de réduire au minimum les pressions d'interface entre son corps et la surface du matelas.

L'évaluation ou la mesure des pressions d'interface est notamment réalisée par l'intermédiaire de capteurs de technologies diverses disposés généralement sous les cellules gonflables des matelas sur lesquels sont allongés les patients.

Ces capteurs permettent selon leur technologie de déterminer soit la ligne de flottaison, c'est-à-dire la distance d'enfoncement du corps des patients sur les cellules gonflables de leur dispositif de support, soit les pressions appliquées par le corps des patients sur les cellules gonflables de leur dispositif de support.

En fonction du signal de réponse du capteur la profondeur d'enfoncement ou la pression appliquée par le corps sur le dispositif de support est déterminée par un dispositif électronique de commande et de régulation et comparée à des valeurs de consigne prédéterminée en fonction de la morphologie du patient. Lorsque la profondeur ou la pression calculée à partir du signal de réponse du capteur se situe hors de la plage de valeurs de consigne le dispositif électronique de commande et de régulation actionne des moyens de gonflage pour ajuster la pression de gonflage des cellules gonflables du dispositif de support pour assurer un support et un confort adaptés à la morphologie et la position du patient.

Comme indiqué précédemment il existe à ce jour différents capteurs qui ont été spécifiquement développés pour réguler la pression de gonflage de dispositifs de support à cellules gonflables comme les matelas thérapeutiques.

On peut citer en particulier les documents FR-A-2757378 et WO-A-9939613 au nom de la demanderesse, qui décrivent respectivement deux capteurs de technologies distinctes.

Le document FR-A-2757378 décrit un dispositif de support à cellules gonflables comportant un dispositif de commande comprenant un capteur inductif disposé sous les cellules gonflables du matelas au niveau de la zone sacrée du patient. Le capteur inductif permet de mesurer une distance d'enfoncement du corps d'un patient allongé sur le dispositif de support comportant le capteur et de commander des moyens de gonflage des cellules pour réaliser une régulation de la pression de gonflage des cellules en fonction de la distance d'enfoncement mesurée par le capteur.

Un premier inconvénient d'un tel capteur inductif résulte de son coût de fabrication élevé qui impacte fortement le coût du dispositif de support lui-même. Par ailleurs, un tel capteur inductif présente une épaisseur d'au moins 5cm ce qui implique une épaisseur importante du dispositif de support l'intégrant, épaisseur rendant plus difficile la réalisation d'une couverture sûre par les barrières de sécurité installées sur le châssis du dispositif de support pour prévenir les chutes.

Enfin, il convient d'assurer la satisfaction d'un tel capteur inductif aux exigences normatives en matière de compatibilité électromagnétique (CEM) car c'est un capteur à champs électromagnétique.

Le document WO-A-9939613 présente lui aussi un dispositif de support à cellules gonflables pour supporter le corps d'un patient. Ce dispositif de support comporte un capteur de pression comprenant lui-même une chambre gonflable gonflée à une pression prédéterminée. Le capteur est logé sous les cellules gonflables de la zone sacrée de support du patient. Ainsi la pression interne de la chambre gonflable du capteur varie suivant les variations de pression dans les cellules gonflables du dispositif de support en fonction de la morphologie, de la masse et des mouvements du patient reposant sur le dispositif de support. Des moyens électroniques comparent ensuite les pressions respectives dans la chambre gonflable du capteur et dans les cellules gonflables du dispositif de support et commandent en conséquence des moyens de gonflage pour réguler et ajuster la pression de gonflage à l'intérieur des cellules lorsque la comparaison des pressions se situe hors d'une plage de valeurs de consigne prédéterminée. Afin d'assurer sa fonction de capteur, la capteur est rempli d'un fluide faiblement compressible, par exemple de l'huile de silicone.

Un inconvénient d'un tel capteur hydraulique réside, comme pour les capteurs inductifs, dans son coût de fabrication élevé qui impacte fortement le coût du dispositif de support lui-même. Par ailleurs, un tel capteur hydraulique présente également une épaisseur d'au moins 3 cm ce qui implique une épaisseur importante du dispositif de support l'intégrant, épaisseur rendant plus difficile la réalisation d'une couverture sûre par les barrières de sécurité installées sur le châssis du dispositif de support pour prévenir les chutes.

Enfin, un tel capteur à chambre gonflable est lourd car il contient environ 4 kg d'huile silicone, ce qui rend ses manipulations délicates.

Les différents capteurs et systèmes associés de régulation de la pression de gonflage des cellules de matelas et autres dispositifs de support à usage thérapeutique ont pour principal problème leur coût de fabrication et de mise en oeuvre qui limite leur utilisation aux dispositifs de support employés et exploités dans les hôpitaux, en particulier au sein de services spécialisés pour la prise en charge et le traitement de patients à mobilité très réduite et/ou à fort risques de développement d'escarres.

De plus, les capteurs connus de l'art antérieur ont une structure propre généralement complexe et ils nécessitent des interfaces et systèmes électroniques d'exploitation des mesures également sophistiqués, qui sont donc non seulement chers mais également difficiles à entretenir et le cas échéant modifier ou remplacer en cas de défaillances et/ou de disfonctionnement. Il convient alors de changer l'intégralité de dispositif de support et non simplement l'élément de celui-ci défaillant, ce qui est pénible pour les patients et particulièrement coûteux pour les hôpitaux.

Enfin, le problème de coût important des capteurs et systèmes de régulation de pression des dispositifs de support à cellules gonflables existants interdit à ce jour pour une grande majorité de patients l'exploitation de tels dispositifs de support dans le cadre d'une médicalisation à domicile car les organismes de sécurité sociale et autres mutuelles refusent généralement la prise en charge et le remboursement des coûts inhérents à l'achat et l'utilisation de tels dispositifs de support à cellules gonflables.

Il existe donc un problème technique important consistant à concevoir et réaliser un capteur de pression apte à être utilisé dans des dispositifs de support à cellules gonflables, et en particulier des dispositifs de type matelas ou coussin thérapeutique, pour permettre une régulation de pression à l'intérieur des cellules qui présente un coût de revient très inférieur aux capteurs et systèmes existants tout en procurant des performances similaires. Un tel capteur est notamment nécessaire pour permettre une réduction du coût de ces dispositifs de support et ainsi leur achat et utilisation par des utilisateurs dans le cadre d'une médicalisation à domicile.

Un second problème technique consiste également à réaliser un capteur apte à être utilisé dans des dispositifs de support à cellules gonflables pour permettre une régulation de pression à l'intérieur des cellules qui puisse être rapidement et facilement remplacé en cas de panne ou de disfonctionnement au sein des systèmes de régulations des dispositifs de support à cellules gonflables existants.

Dans US-6 386 051 on décrit l'utilisation d'une pluralité notamment de plus de 200 capteurs, à cellules résistives FSR organisées en réseaux sur toute la surface supérieure d'un matelas, c'est-à-dire par-dessus le matelas, en vue de déterminer la posture d'un patient reposant sur le matelas. Dans US-2006/0070456, on décrit l'utilisation là encore d'une pluralité de capteurs (FSR) appliqués sur la presque totalités d'un châssis dur de lit uniquement à des fins de détection de la présence du patient sur le lit à titre d'alarme.

Ces types de capteurs FSR sont également mis en oeuvre classiquement dans les sièges automobiles, tels que décrits dans EP 1 821 088. Toutefois, là encore il ne s'agit que de détecter l'occupation du siège par un individu, éventuellement en en déterminant le poids approximatif.

Dans les applications visées dans ces brevets antérieurs, les capteurs ne requièrent pas de fournir un signal analogique avec une réponse sensible et adaptée comprenant une zone de linéarité ou proportionnalité du signal en fonction de la charge exercée sur le capteur. On recherche essentiellement une réponse logique (OUI/NON), c'est-à-dire par contact/absence de contact sur le capteur.

En revanche, selon la présente invention, on cherche à pouvoir réguler le gonflage des cellules d'un matelas à cellules gonflables sur lequel repose le patient en fonction de la charge détectée par le capteur et notamment par un seul capteur localisé au niveau d'une seule zone de matelas, le cas échéant, notamment la zone du sacrum.

Selon la présente invention on doit donc pouvoir à partir des données d'enfoncement des patients dans le matelas au niveau de ladite zone, fournie par le capteur en combinaison avec la mesure de la pression interne du matelas, en déduire le profil d'enfoncement du patient sur toute la surface du matelas.

D'autre part, selon la présente invention le capteur doit être adapté pour fournir des réponses exploitables tout en étant disposées sous le matelas pour éviter le contact du capteur avec le patient, ce qui pourrait être cause d'escarres et notamment le capteur selon l'invention doit pouvoir être disposé entre deux couches de matelas, sous une couche supérieure constituée de cellules gonflables.

Pour ce faire, on cherche à fournir un capteur qui puisse donner une réponse la plus adaptée en termes de sensibilité d'un signal analogique, présentant une zone de proportionnalité du signal en fonction de la charge exercée sur le capteur, pour des charges de l'ordre de 40 à 210 Kg (poids d'un individu).

En effet, l'impédance d'une cellule FSR est variable en fonction de la force de compression au cm² qui s'exerce sur elle, mais sa courbe de réponse en fonction de la force appliquée est en général non linéaire. Il y a donc de très fortes variations de résistance sur de faibles contraintes et à l'inverse, plus les contraintes sont importantes, plus les variations de résistance sont moindres.

Ce qui rend difficile l'obtention d'un signal analogique, c'est-à-dire proportionnel à la charge exercée.

Une solution à ces problèmes réside selon la présente invention dans un capteur de pression, notamment pour détecter et mesurer au moins une pression d'appui appliquée sur une structure de support, caractérisé en ce qu'il comporte au moins une cellule résistive de détection de forces, appliquée sur une semelle rigide, ladite semelle étant logée dans une réservation formée dans une première couche intermédiaire compressible, de préférence une réservation ou découpe de forme complémentaire au contour de ladite semelle disposée entre au moins deux plaques protectrices. Les deux plaques protectrices permettent d'éviter tout appui ponctuel ou tranchant direct sur une surface de ladite cellule résistive de détection de forces et contribuent à convertir un tel appui en un appui uniforme sur toute la surface de ladite cellule.

La rigidité ou semi-rigidité de ces plaques de protection est telle qu'elles ne subissent pas de déformation en flexion sous l'effet d'une charge correspondant à celle d'un adulte de 40 à 210 Kg.

Dans un mode préféré de réalisation, le capteur de pression comporte au moins une seconde couche intermédiaire en matériau compressible, disposée entre une dite plaque protectrice et ladite cellule résistive et en contact avec celles-ci.

On comprend que ladite seconde couche intermédiaire compressible est ainsi en contact avec ladite cellule résistive.

Le capteur de pression de l'invention est parfaitement adapté à la détection et la mesure de pressions et de variations de pressions dans un dispositif de support à cellules gonflables. En effet, lorsque le capteur est inséré sous les cellules gonflables d'un dispositif de support comme dans l'art antérieur avec lesdites plaques de protection rigides comme plaques supérieure et inférieure, le poids du corps d'un patient allongé sur les cellules gonflables d'un tel dispositif de support engendre l'application de forces par les cellules gonflables sur le capteur de l'invention ce qui provoque une variation d'impédance de la ou des cellules résistives de détection de forces à l'intérieur du capteur, qui engendre alors une variation du signal électrique mesuré aux bornes du capteur.

Les variations du signal électrique aux bornes du capteur de l'invention sont donc proportionnelles aux déplacements du corps du patient sur les cellules gonflables du dispositif de support et aux variations de pressions hydrauliques dans les cellules gonflables consécutives à ces déplacements. il est donc possible d'exploiter le signal électrique aux bornes du capteur pour contrôler et réguler la pression de gonflage des cellules du dispositif de support notamment à l'aide d'un système de commande et de régulation de la pression de gonflage de cellules d'un dispositif de support tel qu'un matelas à air similaire à celui précédemment décrit par le déposant dans le brevet US 6, 009, 580.

Le capteur de l'invention est également singulier et particulièrement avantageux en ce qu'il comporte et utilise des cellules résistives de détection de force. Ces cellules, plus connues sous leur dénomination anglaise "Force Sensing Resistors" ou FSR (marque déposée) présentent une structure extrêmement simple constituée, dans leur forme la plus simple de deux feuilles de polymères laminées ensemble, l'une des feuilles étant recouverte d'un réseau d'électrodes à plages intercalées, constituées par exemple de cuivre ou d'argent, et l'autre feuille étant recouverte d'un matériau résistif tel qu'une encre ou un matériau semi-conducteur par exemple.

La relative simplicité de construction des cellules résistives de détection de force, qui sont produites et exploitées en série pour un grand nombre d'application diverses confère à ces cellules un prix particulièrement faible en comparaison d'autres composants électroniques ce qui confère au capteur de pression de l'invention un prix de revient extrêmement réduit en comparaison de celui des capteurs connus de l'art antérieur.

De plus, la structure du capteur de l'invention n'en est que plus simple et aisée à remplacer ou réparer en cas de défaillance du capteur.

Par ailleurs, les cellules résistives de détection de force, qui sont des composants électroniques dont l'impédance décroit d'autant plus que l'intensité d'une force appliquée perpendiculairement sur leur surface augmente, sont peu sensibles aux bruits et aux vibrations ce qui facilite la réalisation et l'exploitation des mesures. Leur large plage d'impédance permet également l'emploi d'une électronique d'interface et d'exploitation simplifiée par rapport aux autres capteurs de forces connus à ce jour que sont les extensomètres et les capteurs piézoélectriques, de même que les systèmes électroniques d'exploitation des capteurs de pressions ou de mesure d'enfoncement jusqu'à présent employés dans des dispositifs de support à cellules gonflables tels que des matelas et coussins thérapeutiques.

De plus, les cellules résistives de détection de forces étant particulièrement minces (de 0,20 à 0,75 mm), insensibles à la température, aux produits chimiques et à l'humidité, le capteur de l'invention s'avère également beaucoup plus robuste et résistant que les capteurs de pression de l'art antérieur, tout en procurant une précision et une fidélité de mesure au moins aussi bonnes sinon meilleures que les capteurs connus beaucoup plus onéreux. En effet, l'impédance du capteur de l'invention varie de façon linéaire suivant une courbe d'équation Z(Ω)=α x P(bar) dans un plage d'impédance comprise entre 500 Ω et 20 kΩ.

En ce qui concerne les couches intermédiaires par « compressible », on entend que lesdites couches sont compressibles par pression sur une ou les deux plaques protectrices entre lesquelles elles sont intercalées.

On comprend donc que la ou lesdites couches intermédiaires sont moins rigides que les plaques protectrices.

Comme mentionné ci-dessus, ladite cellule résistive de détection de forces est plaquée sur un support rigide entre les deux couches protectrices. Un tel support rigide est notamment avantageux pour éviter toute déformation de la ou des cellules résistives de détection de force du capteur lorsque celui-ci est mis en place sous les cellules gonflables d'un dispositif de support. De telles déformations des cellules résistives sont en effet à bannir car elles entraînent des variations d'impédance des cellules non liées aux forces que l'on souhaite mesurer par le capteur, c'est-à-dire les forces liées aux déplacements du corps d'un patient allongé sur les cellules gonflables d'un dispositif de support. Aussi est-il avantageux de procurer aux cellules résistives du capteur une semelle rigide empêchant des telles déformations des cellules résistives.

De façon avantageuse, ladite cellule résistive de détection de force est logée dans une réservation formée dans une couche intermédiaire compressible disposée entre les couches protectrices. Cette caractéristique permet de protéger la cellule résistive et de réduire la sensibilité du capteur pour que les mesures réalisées correspondent effectivement à des forces de pression importantes et significatives de mouvements d'un patient allongé sur un dispositif de support à cellules gonflables.

La première couche intermédiaire comprenant des réservations ou découpes dans lesquelles sont logées les semelles et cellules résistives sert à récupérer l'épaisseur de la cellule sur toute la surface des plaques de protection et forme également, le cas échéant, une entretoise entre les différentes cellules en cas de pluralité de cellules dans une pluralité de réservations.

Les deux couches intermédiaires en matériau compressible amortissent les forces de pression appliquées sur le capteur et perçue par la cellule FSR, et contribuent ainsi à homogénéiser les forces de pression appliquées sur le capteur à la surface de la ou des cellule(s) FSR afin d'adapter la sensibilité et la linéarité du signal de réponse de la cellule en fonction des forces appliquées.

Les deux plaques protectrices permettent d'éviter tout appui ponctuel ou tranchant direct sur une surface de ladite cellule résistive de détection de forces et contribuent aussi à convertir un tel appui en un appui uniforme sur toute la surface de ladite cellule.

Mais, surtout, cette configuration permet d'ajuster la dynamique de réponse du signal en fonction des matériaux choisis et de leur dimensionnement, notamment en épaisseur.

Ces couches intermédiaires sont effectivement dimensionnées pour permettre d'obtenir une réponse sensiblement linéaire du capteur dans une plage de charge correspondant à la masse d'un individu de 40 à 210 Kg.

Toujours selon l'invention, lesdites plaques protectrices sont constituées d'un matériau rigide, notamment un matériau métallique ou synthétique. En variante, et toujours selon l'invention, les plaques protectrices peuvent également être constituées d'un matériau synthétique expansé, mais dont la densité confère une certaine rigidité ou semi-rigidité.

De préférence, une dite couche intermédiaire est formée d'au moins une couche de matériau compressible apte à subir une réduction d'épaisseur d'au plus 40% sous une pression comprise entre 100 et 250 N/cm².

On considère en effet que pour obtenir une réponse linéaire fiable et reproductible du capteur il convient que l'écrasement maximal de la couche compressible du capteur soit de l'ordre de 40 %.

Dans un premier mode de réalisation du capteur de l'invention, une dite couche intermédiaire est constituée d'une mousse de matériau synthétique, de préférence d'une mousse de polyéthylène et d'une épaisseur comprise entre 3 et 30 mm environ.

En particulier, la mousse des couches intermédiaires présente une densité de 35 à 65 kg/m³.

L'épaisseur de la couche intermédiaire peut notamment varier selon l'application et peut même aller au-delà de 30 mm dans certains cas.

Dans un autre mode de réalisation du capteur de l'invention, ladite couche intermédiaire est constituée d'un matériau composite, tissé et/ou structuré en trois dimensions, en particulier composé de polyester tissé. De tels matériaux composites tissés en trois dimensions sont notamment fabriqués et commercialisés par la société JOHN HEATCOAT & COMPANY LIMITED, enregistrée au Royaume-Uni.

Conformément à une autre caractéristique préférée de l'invention, ladite semelle rigide de au moins ladite cellule résistive de détection de forces, est constitué d'une plaque métallique, notamment à base d'aluminium ou encore, en variante, d'une plaque de résine, notamment de résine Epoxy.

Dans une autre forme de réalisation du capteur de l'invention, particulièrement ingénieuse, ladite cellule résistive de détection de forces est constituée par un circuit imprimé sur une plaque rigide, constituant ladite semelle en particulier sur une plaque de résine Epoxy. Cette forme de réalisation particulière de la cellule résistive constitue le niveau d'intégration et de compacité maximum que l'on peut envisager pour le capteur de l'invention, même si sa réalisation est plus coûteuse.

Toujours selon l'invention, il est également préférable que le capteur de l'invention comporte une pluralité de cellules résistives de détection de forces, lesquelles peuvent être disposées en réseau et uniformément réparties entre lesdites couches protectrices et étant connectées en série ou en parallèle. Dans ce mode de réalisation, on augmente non seulement la précision de détection et de mesure du capteur mais également si on le souhaite sa surface de détection et de mesure et par là même on augmente la résistance de la mesure à la distorsion parasite.

Un autre objet de l'invention concerne un dispositif de support, notamment un matelas ou un coussin, d'un élément à supporter, notamment le corps d'un patient, comportant au moins une couche supérieure reposant sur au moins une couche inférieure de support de ladite couche supérieure, lesdites couches supérieure et inférieure étant, de préférence, intégrées dans une housse, de préférence amovible, Le dispositif de support de l'invention se caractérise par le fait qu'il comporte un capteur de pression tel que défini précédemment disposé entre la couche supérieure et la couche inférieure et relié à un dispositif de commande et de régulation pour détecter les pressions appliquées par l'élément à supporter sur ladite couche supérieure du dispositif de support et déterminer leur valeur et leur localisation.

Dans un mode de réalisation préférée du dispositif de support de l'invention, la couche supérieure est constituée de cellules gonflables d'un fluide, en particulier d'air, et lesdites cellules communiquent hydrauliquement de préférence pneumatiquement entre elles et avec des moyens de gonflage aptes à être pilotés par le dispositif de commande du dispositif de support pour remplir ou vider les cellules gonflable de la couche supérieure du matelas, en fonction des pressions détectées et mesurées par le capteur sur ladite couche supérieure.

Par ailleurs, dans un autre mode de réalisation préféré du dispositif de support de l'invention, la couche inférieure est, elle aussi, constituée de cellules gonflables d'un fluide, en particulier d'air et communiquant avec des moyens de gonflage reliés et aptes à être pilotés par le dispositif de commande pour remplir ou vider les cellules de la couche inférieure, en fonction des pressions détectées et mesurées par ledit capteur et/ou de la pression de gonflage des cellules de la couche supérieure du dispositif de support.

Selon un autre mode de réalisation préféré du dispositif de support de l'invention, la couche supérieure et la couche inférieure communiquent hydrauliquement entre elles de manière à être à la même pression.

Un dernier objet de l'invention concerne également un procédé de régulation du gonflage d'un dispositif de support selon l'invention, notamment un matelas ou un coussin, à l'aide d'un capteur selon l'invention, ledit capteur étant disposé dans ledit dispositif de support selon l'invention ou sous celui-ci tel que défini dans la revendication 18.

Selon ce procédé, ledit dispositif de support comporte des cellules gonflables d'un fluide, en particulier d'air, communiquant hydrauliquement de préférence pneumatiquement avec des moyens de gonflage desdites cellules pilotés par un dispositif de commande est relié électriquement au capteur de pression, de manière à gonfler, respectivement, dégonfler lesdites cellules gonflables du dispositif de support, en fonction des pressions détectées et mesurées par ledit capteur.

Ce procédé est tout particulièrement avantageux en ce qu'il permet entre autres de réaliser une régulation de la pression de gonflage de cellules à air d'un dispositif de support tel qu'un matelas thérapeutique en fonction de variations d'impédances des cellules résistives de détection de forces, qui sont des variations à la fois très simples à détecter et mesurer et très simple à exploiter.

De plus, le capteur de l'invention étant également sensible aux forces de déplacement latérales il permet également lorsqu'il est associé à un dispositif de commande et de régulation approprié de réaliser un suivi des mouvements des patients sur leur matelas gonflable ainsi que des analyses en trois dimensions de la position des patients sur les cellules gonflables de leur matelas pour ajuster en conséquence la pression de gonflage des cellules.

D'autres caractéristiques et avantages ressortiront de la description détaillée qui va suivre de différentes variantes de réalisation du dispositif de support de la présente invention, description réalisée en référence aux figures annexées parmi lesquelles :
- la figure 1A représente schématiquement en perspective et en éclaté le capteur de pression de l'invention dans un premier mode préféré de réalisation ;
- la figure 1B représente schématiquement en perspective et en éclaté le capteur de pression de l'invention dans un second mode préféré de réalisation;
- la figure 2 représente schématiquement en perspective et coupe partielle le capteur de pression de l'invention dans un troisième mode préféré de réalisation ;
- la figure 3 représente un mode de réalisation d'un capteur avec quatre cellules résistives disposées en carré ; et
- la figure 4 représente en perspective et en éclaté le dispositif de support selon la présente invention intégrant un capteur de pression conforme à la présente invention.

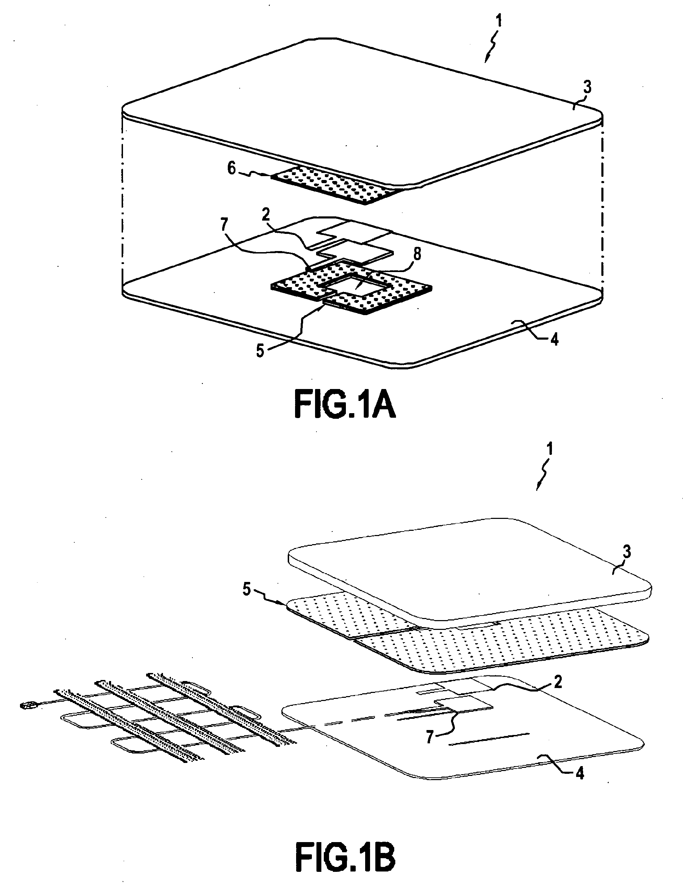

La figure 1A représente un capteur de pressions selon l'invention désigné par la référence générale 1 dans sa forme de réalisation préférée la plus simple.

Le capteur 1 comporte en premier lieu une cellule résistive de détection de forces 2, également nommée par la suite dans la présente description cellule FSR. De telles cellules FSR, notamment produites et commercialisées par exemple par la société FORCE IMAGING TECHNOLOGIES, Inc., sont bien connues de l'homme de l'art et pour cette raison ne seront pas décrites plus en détail dans la présente description.

La cellule FSR 2 est disposée, "en sandwich", entre deux plaques 3, 4 de protection rigides ou semi-rigides. Les plaques 3,4 de protection de la cellule 2 peuvent notamment être constituées d'un matériau thermoplastique tel que le chlorure de polyvinyle (PVC), polypropylène (PP) Polyéthylène (PE) ou encore d'Acrylonitrile Butadiène Styrène (ABS).

En variante, les plaques 3, 4 de protection de la cellule FSR 2 du capteur 1 peuvent également être constituées de métal, notamment d'aluminium par exemple.

La cellule FSR 2 est par ailleurs de façon avantageuse plaquée, par exemple par collage, sur un support rigide 7 constitué par exemple d'une plaque d'aluminium ou de résine Epoxy et logée dans une réservation 8 pratiquée dans une couche 5 de matériau absorbant et compressible collée sur la plaque 4 de rigidification.

Une seconde couche 6 de matériau absorbant et compressible est également collée sur la face interne de la plaque 3.

Le support rigide 7 de la cellule 2 représente une surface de référence pour la mesure de forces de pression par le capteur. En effet, la cellule FSR 2 est sensible à la moindre compression ponctuelle donc aux compressions parasites et autres torsions ou flexions dues à un mauvais état de surface du support sur lequel elle ou le capteur lui-même est appliqué. Il est donc nécessaire de procurer à la cellule une semelle gommant et intégrant les éventuels défauts de la surface sur laquelle le capteur sera implanté pour réaliser des mesures pression.

De la même manière et pour les mêmes raisons, ces couches 5, 6 forment des couches d'interface entre la cellule et les plaques de protection 3, 4. Ces couches 5, 6 peuvent notamment être constitué de mousse synthétique (par exemple de Polyuréthane ou de Polyéthylène) ou d'un matériau synthétique tissé en trois dimensions, notamment de polyester tissé en trois dimensions comme le matériau Spacetec® fabriqué et commercialisé par la société John Heathcoat & Co Ltd, enregistrée au Royaume-Uni.

Ces couches 5, 6 ont également pour fonction de densifier l'espace entre les plaques protectrices 3 et 4 de façon à amortir d'une part les forces de pressions appliquées sur le capteur 1 et perçues par la cellules FSR 2 et d'autre part à réduire l'intensité des forces de pressions perçues par la cellule FSR 2 et homogénéiser la répartition des forces de pression appliquées sur le capteur à la surface de la cellule FSR 2 afin d'en améliorer la sensibilité et la linéarité du signal de réponse de la cellule en fonction des forces appliquées.

En effet, l'impédance de la cellule FSR 2 est variable en fonction de la force de compression au centimètre carré qui s'exerce sur elle et sa courbe de réponse en fonction de la force appliquée est non linéaire. Il y a donc de très fortes variations de résistance sur de faibles contraintes et à l'inverse, plus les contraintes sont importantes plus les variations de résistances sont moindres.

De préférence, les couches 5, 6 sont notamment dimensionnées pour obtenir une réponse linéaire du capteur dans une plage de pressions appliquées sur un dispositif de support à cellules gonflables par le corps de personnes d'une masse de 40 à 210 kg.

Ainsi le capteur 1 de la présente invention est donc un complexe formé d'une cellule FSR 2 appliquée sur une semelle 7, encapsulée avec deux couches intermédiaires 5, 6 de matériaux absorbant et compressible entre deux plaques 3, 4 de protection rigide ou semi-rigide, lesquelles sont de préférence jointes l'une à l'autre par des moyens de liaison réversibles appropriés.

Par ailleurs, la nature et les caractéristiques de la cellule 2 et des matériaux constitutifs des couches protectrices 3, 4 et des couches intermédiaires 5, 6 sont choisis de sorte que le signal de réponse du capteur 1 soit proportionnel aux forces de pressions que le capteur doit être capable de détecter et mesurer en fonction de l'application dans laquelle il doit être utilisé. En particulier, dans le cadre d'une utilisation aux fins de régulation de la pression de gonflage de cellules gonflables d'un dispositif de support médicalisé tel qu'un matelas thérapeutique, le capteur 1 est conçu pour procurer un signal de réponse sensiblement proportionnel au poids des patients.

La figure 1B représente une variante de réalisation du capteur 1 de la figure 1A dans laquelle les deux couches intermédiaires 5, 6 sont remplacées par une couche intermédiaire unique 5 de format sensiblement identique à celui des couches protectrices 3, 4 et d'épaisseur sensiblement égale à l'épaisseur totale des deux couches 5, 6 dans le mode de réalisation de la figure 1A ou légèrement inférieure. La cellule FSR 2 et sa semelle 7 sont alors logées dans une réservation de forme complémentaire réalisée par découpe dans la couche intermédiaire 5.

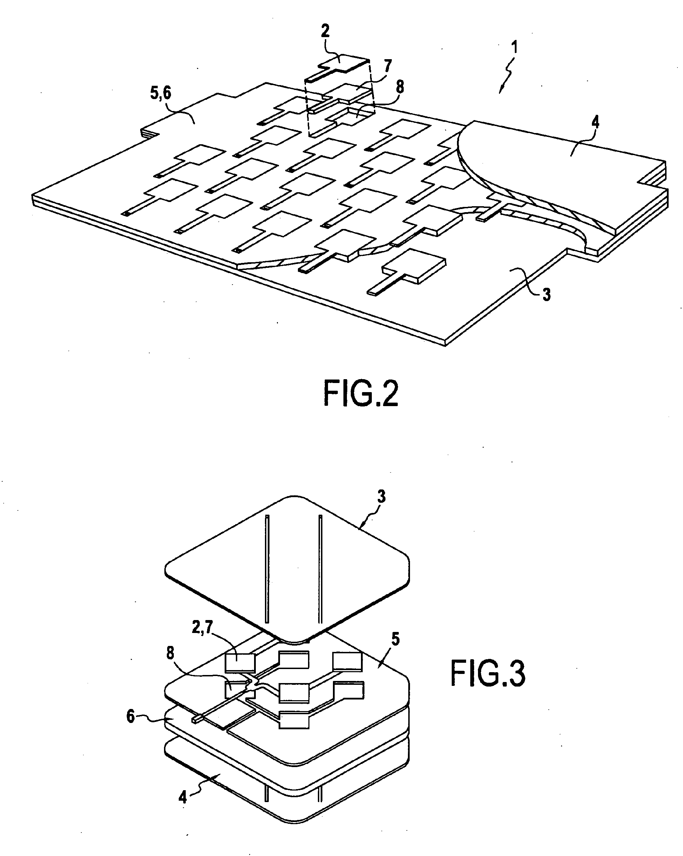

La figure 2 représente un troisième mode de réalisation préféré du capteur de pression 1 de l'invention. Sur cette figure les éléments de constitution et de fonction similaire ou identique à ceux du capteur des figures 1A et 1B portent les mêmes références numériques.

Dans ce mode de réalisation, le capteur 1 comporte une pluralité de cellules FSR 2. Ces cellules FSR 2, dont le nombre peut varier de 1 à 25 de préférence en fonction de la surface du capteur 1 que l'on veut réaliser, sont chacune collées sur un support ou semelle rigide 7, par exemple une plaque d'aluminium ou de résine Epoxy, et disposées régulièrement et uniformément en quinconce dans des réservations 8 de forme complémentaire du contour des cellules 2 et de leur plaque support 7.

La deuxième couche intermédiaire de matériau compressible 6 est appliquée du côté de la cellule résistive et non pas du côté de la semelle rigide et est en contact avec la cellule résistive.

L'épaisseur de la première couche intermédiaire 5, et donc l'épaisseur des réservations 8, est sensiblement identique à l'épaisseur cumulée des cellules 2 et semelles rigides.

Les réservations 8 de logement des cellules FSR 2 sont formées par des découpes dans une couche intermédiaire 5, par exemple une mousse synthétique ou une couche de matériau synthétique tissé en trois dimensions, notamment de polyester tissé en trois dimensions comme le matériau Spacetec® fabriqué et commercialisé par la société John Heathcoat & Co Ltd, enregistrée au Royaume-Uni.

Sur les figures 1A et 3, lorsque les couches intermédiaires sont constituées de mousse synthétique, de préférence, la première couche intermédiaire 5 est une mousse plus dense notamment avec une densité de 65 kg/m³ et moins épaisse que la mousse de la deuxième couche intermédiaire 6 dont la densité est par exemple de 35 kg/m³, le ratio des épaisseurs des première et deuxième couches intermédiaires étant par exemple de 4 pour 10.

La couche intermédiaire 5 forme ainsi une entretoise entre chacune des cellules FSR 2, dont l'épaisseur est choisie en fonction de la sensibilité et de la précision que l'on souhaite procurer au capteur, ladite couche intermédiaire 5 remplaçant, au moins partiellement, les deux couches intermédiaires 5, 6 du capteur de la figure.

Par ailleurs, la couche intermédiaire 5 est elle-même apposée et collée sur une première couche protectrice 4 et recouverte d'une seconde couche protectrice 3, constituées toutes deux de mousse synthétique, et de préférence de mousse de polyéthylène.

Le capteur 1 représenté à la figure 2, que nous appellerons "multi-cellules" est particulièrement intéressant en ce que la présence d'entretoises entre chaque cellule FSR 2 permet de modifier la réponse du capteur 1 aux contraintes de pressions qui lui sont appliquées et qu'il perçoit. Cette possibilité permet d'ajuster parfaitement la dynamique de la réponse en fonction des densités des matériaux utilisés pour réaliser les couches protectrices 3, 4 ou les entretoises 5 du capteur 1.

Ce capteur multi-cellules permet d'augmenter la surface de lecture et sa précision et surtout, dans le cadre d'un capteur de pressions donnant une réponse tridimensionnelle comme évoqué ci-après, de mieux apprécier la position du patient dans le matelas.

Dans le mode de réalisation de la figure 2, le capteur 1 peut être configuré pour délivrer autant de signaux de sortie que de cellules FSR 2 ou bien un signal unique. Dans le premier cas, le capteur 1 comporte alors un port de connexion (non représenté) au niveau du quel sont rassemblées les bornes de connexion de chacune des cellules FSR 2.

Dans le second cas, les cellules FSR 2 du capteur sont alors connectées entre elles, en série ou en parallèle, pour former un circuit de mesure fermé présentant deux bornes uniques auxquelles le signal de sortie du capteur peut être mesuré.

Par ailleurs, il est également possible dans ce mode de réalisation de connecter les cellules FSR 2 du capteur de manière à pouvoir réaliser des mesures en trois dimensions afin de mesurer non seulement l'intensité des forces de pression appliquées sur le capteur mais également leur position d'application sur le capteur 1.

En effet, en lisant chaque cellule FSR individuellement, on peut savoir quelle cellule du capteur subit la contrainte. Ainsi, dans le cadre de l'utilisation d'un tel capteur à la détection de pression dans un dispositif de support thérapeutique, notamment à cellules gonflables, on peut donc détecter où se trouve le malade sur le dispositif de support en détectant la pression appliqués par son corps et sa localisation à l'aide du capteur de l'invention. Dans l'art antérieur on ne détectait que le patient tombait d'un lit qu'une fois qu'il était tombé. Maintenant, il est possible de prévenir la chute puisque l'on peut détecter quand le malade est au bord du matelas. Ceci permet aussi de détecter si le malade est agité.

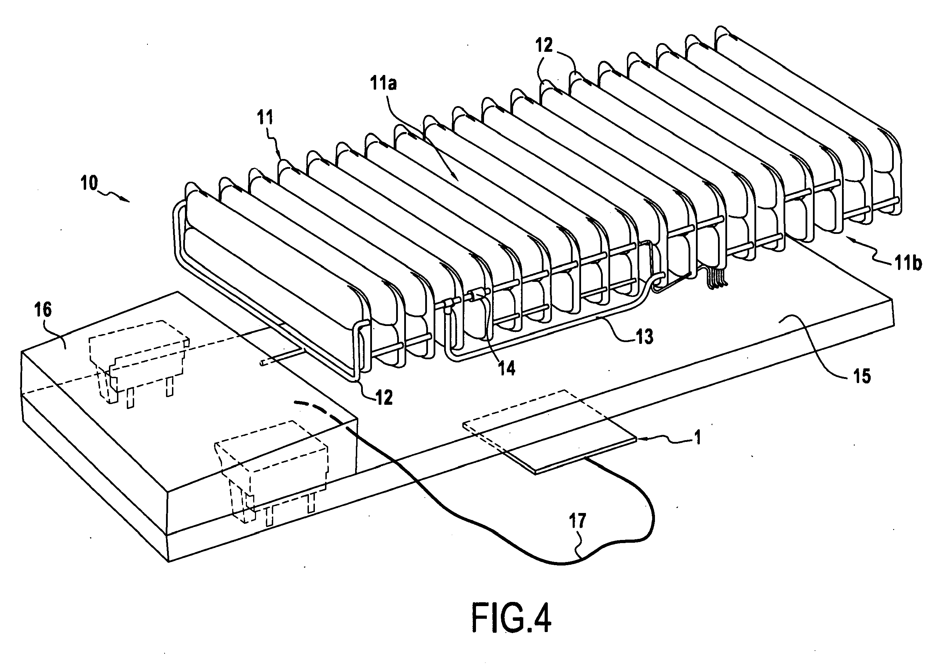

Le capteur de pressions 1 de l'invention tel que présenté précédemment en référence aux figures 1 et 3 a été notamment développé pour une application à la régulation de la pression de gonflage de cellules gonflables de dispositifs de supports tels que des matelas et coussins thérapeutiques à cellules gonflables, comme il va maintenant être décrit en référence à la figure 4 représentant un dispositif de support 10 à cellules gonflables de type matelas thérapeutique.

Le dispositif de support 10 permet de supporter un élément à supporter, en particulier, le corps d'un patient tel qu'un être humain.

Il comprend un matelas gonflable 11 comprenant au moins une cellule gonflable 12 fermée ou à échappement contrôlé.

De préférence, comme représenté sur la figure 4, le matelas 11 est composé d'une multiplicité de cellules gonflables 12 communiquant entre elles par des tuyaux 13 et vannes 14 de connexion hydraulique.

Les cellules gonflables 12 peuvent être de tout type connu de l'homme de l'art et notamment conçues par soudage à plat de deux feuilles souples de polyuréthane (PUR) ou d'une autre matière thermoplastique appropriée.

Le matelas gonflable 11 présente une face supérieure 11a destinée à recevoir un élément à supporter tel que le corps d'un patient et une face inférieure 11b reposant sur une couche inférieure formant une surface de référence 15, généralement constituée d'un matelas de matériau cellulaire tel que de la mousse de forte densité qui, lui-même, peut reposer directement ou indirectement sur un sommier ici non représenté. En variante, ladite couche inférieure 15 peut elle aussi être constituée de cellules gonflables d'un fluide, en particulier d'air.

Le dispositif de support 10 comprend également un dispositif 16 de commande et de régulation de la pression de remplissage des cellules gonflables 12 du matelas 11 (et le cas échéant des cellules gonflable de la surface inférieure de référence 15).

Ce dispositif 16 de commandes et de régulation est avantageusement disposé dans le prolongement du matelas 11 à une extrémité de celui-ci, sur la couche inférieure 15. Il comporte notamment des moyens de remplissage des cellules 12 du matelas 11 comprenant un compresseur ou une pompe relié par les tuyaux 13 et vannes 14 aux cellules 12 pour les remplir ou vider d'un fluide de remplissage, en particulier d'air.

Le dispositif 16 de commande et de régulation comportent également des moyens électroniques (non représentés) de pilotage des moyens de remplissage asservis à des moyens de mesure de pressions.

Ces moyens de mesure de pressions sont essentiellement de deux types à savoir d'une part des moyens (non représentés) de mesure de la pression de gonflage des cellules 12, notamment des moyens de mesure de pression en ligne montés en série dans les tuyaux de connexion hydraulique des cellules, et d'autre part un capteur de pression 1 conforme à la présente invention dans une forme semblable par exemple à celle décrite précédemment à la figure 1 ou à la figure 2.

Le capteur de pression 1 est logé de préférence sous la surface inférieure 11b du matelas 11 ou sous la surface de référence 15 et est relié par une connexion électrique 17 au dispositif 16 de commande et de régulation.

L'ensemble formé des couches supérieure 11 et inférieure 15, du dispositif 16 de commande et de régulation, des tuyaux 13 et vannes 14 et du capteur 1 est de préférence intégré dans une housse protectrice, de préférence également amovible afin de permettre un accès aisé aux éléments du dispositif de support mais également de permettre un remplacement ou nettoyage aisé en cas de besoin.

Lorsque que le patient est positionné sur le matelas le capteur 1 FSR subit une contrainte de compression et son impédance varie de manière proportionnelle au poids du patient par rapport à sa surface corporelle (kg /cm²). La variation d'impédance tient compte de la morphologie du patient puisque l'information sera différente, par exemple, pour deux individus de même poids n'ayant pas les mêmes largeurs de bassin qui n'ont pas le même enfoncement dans le matelas donc pas les mêmes pressions de régulation.

Cette variation de résistance est prise en compte dans le dispositif de commande et de régulation 16 qui modifie sa consigne de régulation proportionnellement à la variation de résistance. Un comparateur du dispositif de commande et de régulation 16 compare la pression dans les cellules 12 au moyen d'un capteur de pression à la nouvelle consigne. Si la pression est trop importante par rapport à la nouvelle consigne le dispositif de commande et de régulation 16 ouvre son électrovanne de dégonflage. Si la pression est insuffisante par rapport à la nouvelle consigne le dispositif de commande et de régulation 16 valide le démarrage de son système de gonflage.En général, on dispose un unique capteur 1 sous la zone de support du sacrum et on peut en déduire la pression au niveau des autres zones du corps du patient allongé sur le dispositif de support dans la mesure où 80% des individus présente une répartition masse/volume des différentes parties du corps similaire.

Pour les personnes ne rentrant pas dans cette norme moyenne ou afin d'obtenir une réponse encore plus spécifique, on installe alors plusieurs capteurs 1 sous les différentes parties du corps dans le dispositif de support.

Le capteur de la figure 1A est disposé avec la plaque protectrice 3 appliquée contre la deuxième couche intermédiaire 6, comme plaque supérieure.

On obtient des résultats similaires lorsque la plaque protectrice 4 appliquée contre la première couche intermédiaire 5 est la plaque supérieure, comme sur la figure 3.

## Revendications

1. Capteur de pression (1), notamment pour détecter et mesurer au moins une pression d'appui appliquée sur un dispositif de support comportant au moins une cellule résistive de détection de force et une première couche intermédiaire compressible, ladite première couche étant disposée entre au moins deux plaques protectrices (3, 4) rigides ou semi-rigides, ledit capteur étant **caractérisé en ce qu'**il comporte au moins une dite cellule résistive (2) de détection de forces appliquée sur une semelle rigide, ladite semelle (7) étant logée dans une réservation (8) formée dans une première couche intermédiaire (5) compressible, de préférence une réservation (8) de forme complémentaire au contour de ladite semelle.

2. Capteur de pression selon la revendication 1, **caractérisé en ce qu'**il comporte au moins une seconde couche intermédiaire (6) en matériau compressible, disposée entre une dite plaque protectrice (3, 4) et ladite cellule résistive (2) et en contact avec celles-ci.

3. Capteur de pression selon l'une des revendications 1 ou 2, **caractérisé en ce que** lesdites plaques protectrices (3, 4) sont constituées d'un matériau rigide métallique ou synthétique.

4. Capteur de pression selon la revendication 3, **caractérisé en ce que** lesdites plaques protectrices (3, 4) sont constituées d'un matériau plastique choisi parmi le PVC, PP, PE ou ABS.

5. Capteur de pression selon l'une des revendications 1 à 4, **caractérisé en ce que** lesdites plaques protectrices (3, 4) sont constituées d'un matériau synthétique expansé ou cellulaire.

6. Capteur de pression selon l'une des revendications 1 à 5, **caractérisé en ce que** ladite couche intermédiaire (5, 6) est formée d'au moins une couche de matériau compressible apte à subir une réduction d'épaisseur de 40% sous une pression comprise entre 100 et 250 N/cm².

7. Capteur de pression selon l'une des revendications 1 à 6, **caractérisé en ce que** ladite couche intermédiaire (5, 6) est constituée d'un matériau cellulaire, de préférence d'une mousse de polyéthylène et d'une épaisseur comprise entre 3 et 30 mm.

8. Capteur de pression selon l'une des revendications 1 à 6, **caractérisé en ce que** ladite couche intermédiaire (5, 6) comprend un matériau structuré en trois dimensions, de préférence un matériau à base de polyester tissé.

9. Capteur de pression selon l'une des revendications 1 à 8, **caractérisé en ce que** ladite semelle rigide (7) de au moins ladite cellule résistive (2) de détection de forces, est constituée d'une plaque métallique, notamment à base d'aluminium.

10. Capteur de pression selon l'une des revendications 1 à 9, **caractérisé en ce que** ladite semelle rigide (7) de au moins ladite cellule résistive (2) de détection de forces est constituée d'une plaque de résine, notamment de résine Epoxy.

11. Capteur de pression selon l'une des revendications 1 à 10, **caractérisé en ce que**, au moins, ladite cellule résistive (2) de détection de forces est constituée par un circuit imprimé sur une plaque rigide, en particulier sur une plaque de résine Epoxy.

12. Capteur selon l'une des revendications 1 à 11, **caractérisé en ce qu'**il comporte une pluralité de cellules résistives (2) de détection de forces.

13. Capteur de pression selon la revendication 12, **caractérisé en ce qu'**il comporte une pluralité de cellules résistives (2) de détection de forces, disposées en réseau et uniformément réparties entre lesdites plaques protectrices (3, 4), dans une pluralité de réservations dans une dite première couche intermédiaire, lesdites cellules étant connectées en série ou en parallèle.

14. Dispositif de support (10) d'un élément à supporter, notamment le corps d'un patient, comportant au moins une couche supérieure (11) reposant sur au moins une couche inférieure (15) de support de ladite couche supérieure, lesdites couches supérieure et inférieure étant, de préférence, intégrées dans une housse de préférence amovible, **caractérisé en ce qu'**il comporte un capteur (1) de pression tel que défini dans l'une des revendications 1 à 13 disposé sous ladite couche supérieure (11) et/ou ladite couche inférieure (15) et relié à un dispositif (16) de commande et de régulation pour détecter les pressions appliquées par ledit élément à supporter sur ladite couche supérieure (11) du dispositif de support et déterminer leur valeur et éventuellement leur localisation.

15. Dispositif de support selon la revendication 14, **caractérisé en ce que** ladite couche supérieure (11) est constituée de cellules (12) gonflables d'un fluide, en particulier d'air et lesdites cellules (12) communiquent hydrauliquement, de préférence pneumatiquement, entre elles et avec des moyens de gonflage aptes à être pilotés par ledit dispositif (16) de commande pour remplir ou vider lesdites cellules (12) de la couche supérieure du matelas, en fonction des pressions détectées et mesurées par ledit capteur (1) sur ladite couche supérieure.

16. Dispositif de support selon la revendication 15, **caractérisé en ce que** ladite couche inférieure (15) est, elle aussi, constituée de cellules gonflables d'un fluide, en particulier d'air et communiquant avec desdits moyens de gonflage reliés et aptes à être pilotés par ledit dispositif (16) de commande pour remplir ou vider lesdites cellules de la couche inférieure, en fonction des pressions détectées et mesurées par ledit capteur et/ou de la pression de gonflage des cellules de la couche supérieure du dispositif de support.

17. Dispositif de support selon la revendication 15 ou la revendication 16, **caractérisé en ce que** ladite couche supérieure (11) et ladite couche inférieure (15) communiquent hydrauliquement entre elles de manière à être à la même pression.

18. Procédé de régulation du gonflage d'un dispositif de support supportant un objet à supporter, notamment le corps d'un patient, dans lequel :
- on détecte et on mesure lesdites pressions appliquées par l'objet à supporter sur le dispositif de support (10) et éventuellement leur localisation au moyen d'un capteur de pression , ledit dispositif de support comportant des cellules (12) gonflables d'un fluide, en particulier d'air, communiquant hydrauliquement, de préférence pneumatiquement, entre elles et avec des moyens de gonflage desdites cellules pilotées par un dispositif (16) de commande et dans lequel ledit dispositif de commande est relié électriquement au dit capteur (1) de pression, et
- on gonfle ou dégonfle lesdites cellules (12) gonflables en fonction des pressions détectées et mesurées par ledit capteur de pression,
**caractérisé en ce que** ledit capteur est tel que défini dans l'une des revendications 1 à 13 et est disposé sous la couche supérieure et/ou sous la couche inférieure d'un dispositif support selon l'une des revendications 14 à 17.

## Claims

1. A pressure sensor (1), notably for detecting and measuring at least one supporting pressure applied on a supporting device including at least one force detection resistive cell and a first compressible intermediate layer, said first layer being positioned between at least two rigid or semi-rigid protective plates (3, 4), said sensor being **characterized in that** it includes at least one said force detection resistive cell (2) applied on a rigid sole, said sole (7) being housed in a reserved space (8) formed in a first compressible intermediate layer (5), preferably a reserved space (8) with a shape matching the contour of said sole.

2. The pressure sensor according to claim 1, **characterized in that** it includes at least one second intermediate layer (6) in a compressible material, positioned between said protective plates (3, 4) and said resistive cell (2) and in contact with the latter.

3. The pressure sensor according to one of claims 1 or 2, **characterized in that** said protective plates (3, 4) consists of a metal or synthetic rigid material.

4. The pressure sensor according to claim 3, **characterized in that** said protective plates (3, 4) consists of a plastic material selected from PVC, PPE, PE or ABS.

5. The pressure sensor according to one of claims 1 to 4, **characterized in that** said protective plates (3, 4) consists of expansed or cellular synthetic material.

6. The pressure sensor according to one of claims 1 to 5, **characterized in that** said intermediate layer (5, 6) is formed with at least one compressible material layer capable of undergoing a 40% reduction in thickness under a pressure comprised between 100 and 250 N/cm².

7. The pressure sensor according to one of claims 1 to 6, **characterized in that** said intermediate layer (5, 6) consists of a cellular material, preferably a polyethylene foam and with a thickness comprised between 3 and 30 mm.

8. The pressure sensor according to one of claims 1 to 6, **characterized in that** said intermediate layer (5, 6) comprises a material structured in three dimensions, preferably a material based on woven polyester.

9. The pressure sensor according to one of claims 1 to 8, **characterized in that** said rigid sole (7) of at least said forced detection resistive cell (2), consists of a metal plate, notably based on aluminium.

10. The pressure sensor according to one of claims 1 to 9, **characterized in that** said rigid sole (7) of at least said force detection resistive cell (2) consists of a plate of resin, notably of epoxy resin.

11. The pressure sensor according to one of claims 1 to 10, **characterized in that** at least said force detection resistive cell (2) is formed by a printed circuit on a rigid plate, in particular on a plate of epoxy resin.

12. The sensor according to one of claims 1 to 11, **characterized in that** it includes a plurality of resistive cells (2) for detecting forces.

13. The pressure sensor according to claim 12, **characterized in that** it includes a plurality of force detection resistive cells (2), arranged as an array and uniformly distributed between said protective plates (3, 4), in a plurality of reserved spaces in said first intermediate layer, said cells being connected in series or in parallel.

14. A supporting device (10) for an element to be supported, notably the body of a patient, including at least one upper layer (11) resting on at least one lower layer (15) for supporting said upper layer, said upper and lower layers being preferably integrated into a preferably removable cover, **characterized in that** it includes a pressure sensor (1) as defined in one of claims 1 to 13, arranged under said upper layer (11) and/or said lower layer (15) and connected to a control and regulation device (16) for detecting the pressures applied by said element to be supported on said upper layer (11) of the supporting device, and determining their value and optionally their localization.

15. The supporting device according to claim 14, **characterized in that** said upper layer (11) consists of cells (12) inflatable with a fluid, in particular air and said cells (12) hydraulically, preferably pneumatically communicate with themselves and with inflating means capable of being driven by said control device (16) in order to fill or empty said cells (12) of the upper layer of the mattress, depending on the pressure detected and measured by said sensor (1) on said upper layer.

16. The supporting device according to claim 15, **characterized in that** said lower layer (15) also itself consists of cells inflatable with a fluid, in particular air and communicating with said inflating means connected and capable of being driven by said control device (16) in order to fill or empty said cells of the lower layer, depending on the pressures detected and measured by said sensor, and/or on the inflation pressure of the cells of the upper layer of the supporting device.

17. The supporting device according to claim 15 or claims 16, **characterized in that** said upper layer (11) and said lower layer (15) hydraulically communicate with each other so as to be at the same pressure.

18. A method for regulating the inflation of a supporting device supporting an object to be supported, notably the body of a patient, wherein:
- said pressures applied by the object to be supported on the supporting device (10) and optionally their localisation are detected and measured by means of a pressure sensor, said supporting device including cells (12) inflatable with a fluid, in particular air, hydraulically, preferably pneumatically, communicating with each other and with means for inflating said cells driven by a control device (16), and wherein said control device is electrically connected to said pressure sensor (1), and
- said inflatable cells (12) are inflated or deflated depending on the pressure detected and measured by said pressure sensor,
**characterized in that** said sensor is as defined in one of claims 1 to 13, and is positioned under the upper layer and/or under the lower layer of a supporting device according to one of claims 14 to 17.

## Patentansprüche

1. Druckfühler (1), insbesondere zum Erfassen und Messen wenigstens eines auf eine Tragvorrichtung ausgeübten Auflagedruckes, umfassend wenigstens eine Widerstandszelle zur Krafterfassung sowie eine erste komprimierbare Zwischenschicht, wobei die erste Schicht zwischen wenigstens zwei starren oder halbstarren Schutzplatten (3, 4) angeordnet ist, wobei der Fühler **dadurch gekennzeichnet ist, daß** er wenigstens eine Widerstandszelle (2) zur Krafterfassung, die auf eine starre Sohle aufgebracht ist, umfaßt, wobei die Sohle (7) in einer in einer ersten komprimierbaren Zwischenschicht (5) ausgebildeten Aufnahme (8), vorzugsweise einer Aufnahme (8) mit einer zur Kontur der Sohle ergänzenden Form aufgenommen ist.

2. Druckfühler nach Anspruch 1, **dadurch gekennzeichnet, daß** er wenigstens eine zweite Zwischenschicht (6) aus komprimierbarem Material umfaßt, die zwischen einer Schutzplatte (3, 4) und der Widerstandszelle (2) sowie in Kontakt mit diesen angeordnet ist.

3. Druckfühler nach einem der Ansprüche 1 oder 2, **dadurch gekennzeichnet, daß** die Schutzplatten (3, 4) aus einem starren, metallischen oder synthetischen Material bestehen.

4. Druckfühler nach Anspruch 3, **dadurch gekennzeichnet, daß** die Schutzplatten (3, 4) aus einem plastischen Material, das aus PVC, PP, PE oder ABS ausgewählt ist, bestehen.

5. Druckfühler nach einem der Ansprüche 1 bis 4, **dadurch gekennzeichnet, daß** die Schutzplatten (3, 4) aus einem Schaum- oder Zellkunststoff bestehen.

6. Druckfühler nach einem der Ansprüche 1 bis 5, **dadurch gekennzeichnet, daß** die Zwischenschicht (5, 6) von wenigstens einer Schicht aus komprimierbarem Material, das geeignet ist, eine Dickenreduzierung von 40 % unter einem Druck zwischen 100 und 250 N/cm² zu erfahren, gebildet ist.

7. Druckfühler nach einem der Ansprüche 1 bis 6, **dadurch gekennzeichnet, daß** die Zwischenschicht (5, 6) von einem Zellmaterial, vorzugsweise einem Polyethylenschaum gebildet ist und eine Dicke im Bereich zwischen 3 und 30 mm aufweist.

8. Druckfühler nach einem der Ansprüche 1 bis 6, **dadurch gekennzeichnet, daß** die Zwischenschicht (5, 6) ein strukturiertes, dreidimensionales Material, vorzugsweise ein Material auf der Basis von gewebtem Polyester umfaßt.

9. Druckfühler nach einem der Ansprüche 1 bis 8, **dadurch gekennzeichnet, daß** die starre Sohle (7) von wenigstens der Widerstandszelle (2) zur Krafterfassung von einer Metallplatte, insbesondere auf Aluminiumbasis gebildet ist.

10. Druckfühler nach einem der Ansprüche 1 bis 9, **dadurch gekennzeichnet, daß** die starre Sohle (7) von wenigstens der Widerstandszelle (2) zur Krafterfassung von einer Platte aus Harz, insbesondere aus Epoxidharz gebildet ist.

11. Druckfühler nach einem der Ansprüche 1 bis 10, **dadurch gekennzeichnet, daß** wenigstens die Widerstandszelle (2) zur Krafterfassung von einer auf eine starre Platte, insbesondere auf eine Epoxidharzplatte gedruckten Schaltung gebildet ist.

12. Fühler nach einem der Ansprüche 1 bis 11, **dadurch gekennzeichnet, daß** er eine Vielzahl von Widerstandszellen (2) zur Krafterfassung umfaßt.

13. Druckfühler nach Anspruch 12, **dadurch gekennzeichnet, daß** er eine Vielzahl von Widerstandszellen (2) zur Krafterfassung umfaßt, die zwischen den Schutzplatten (3, 4) als Netz angeordnet und in einer Vielzahl von Aufnahmen in einer ersten Zwischenschicht gleichmäßig verteil sind, wobei die Zellen in Reihe oder parallel geschaltet sind.

14. Vorrichtung zum Tragen (10) eines zu tragenden Elements, insbesondere des Körpers eines Patienten, umfassend wenigstens eine obere Schicht (11), die auf wenigstens einer unteren Schicht (15) zum Tragen der oberen Schicht aufliegt, wobei die obere und die untere Schicht vorzugsweise in einen vorzugsweise abnehmbaren Bezug integriert sind, **dadurch gekennzeichnet, daß** sie einen Druckfühler (1), wie er in einem der Ansprüche 1 bis 13 definiert ist, umfaßt, der unter der oberen Schicht (11) und/oder der unteren Schicht (15) angeordnet und mit einer Steuerungs- und Regulierungsvorrichtung (16) verbunden ist, um die durch das zu tragende Element auf die obere Schicht (11) der Tragvorrichtung ausgeübten Drücke zu erfassen und deren Wert sowie eventuell deren Ort zu bestimmen.

15. Tragvorrichtung nach Anspruch 14, **dadurch gekennzeichnet, daß** die obere Schicht (11) von Zellen (12), die mit einem Fluid, insbesondere mit Luft aufblasbar sind, gebildet ist und die Zellen (12) hydraulisch, vorzugsweise pneumatisch untereinander sowie mit Aufblasmitteln in Verbindung stehen, die geeignet sind, über die Steuerungsvorrichtung (16) gesteuert zu werden, um die Zellen (12) der oberen Schicht der Matratze in Abhängigkeit der durch den Fühler (1) an der oberen Schicht erfaßten und gemessenen Drücke zu füllen oder zu entleeren.

16. Tragvorrichtung nach Anspruch 15, **dadurch gekennzeichnet, daß** die untere Schicht (15) ebenfalls von Zellen gebildet ist, die mit einem Fluid, insbesondere mit Luft aufblasbar sind und mit Aufblasmitteln in Verbindung stehen, die verbunden und geeignet sind, über die Steuerungsvorrichtung (16) gesteuert zu werden, um die Zellen der unteren Schicht in Abhängigkeit der durch den Fühler erfaßten und gemessenen Drücke und/oder des Aufblasdruckes der Zellen der oberen Schicht der Tragvorrichtung zu füllen oder zu entleeren.

17. Tragvorrichtung nach Anspruch 15 oder Anspruch 16, **dadurch gekennzeichnet, daß** die obere Schicht (11) und die untere Schicht (15) hydraulisch untereinander in Verbindung stehen, so daß sie auf dem gleichen Druck sind.

18. Verfahren zum Regulieren des Aufblasens einer Tragvorrichtung, die ein zu tragendes Objekt, insbesondere den Körper eines Patienten trägt, wobei:
- die durch das zu tragende Objekt auf die Tragvorrichtung (10) ausgeübten Drücke und eventuell deren Ort mittels eines Druckfühlers erfaßt und gemessen werden, wobei die Tragvorrichtung mit einem Fluid, insbesondere mit Luft aufblasbare Zellen (12) umfaßt, die hydraulisch, vorzugsweise pneumatisch untereinander sowie mit über eine Steuerungsvorrichtung (16) gesteuerten Mitteln zum Aufblasen der Zellen in Verbindung stehen, und wobei die Steuerungsvorrichtung mit dem Druckfühler (1) elektrisch verbunden ist, und
- die aufblasbaren Zellen (12) in Abhängigkeit der durch den Druckfühler erfaßten und gemessenen Drücke aufgeblasen oder entleert werden,
**dadurch gekennzeichnet, daß** der Fühler derart ist, wie in einem der Ansprüche 1 bis 13 definiert, und unter der oberen Schicht und/oder unter der unteren Schicht einer Tragvorrichtung nach einem der Ansprüche 14 bis 17 angeordnet ist.
